# EUROPEAN PATENT APPLICATION

(11) **EP 4 332 216 A1**
(43) Date of publication of application: **06.03.2024**
(21) Application number: 22795884.0
(22) Date of filing: 28.04.2022
(51) Int. Cl.: C12N 5/10, A61K 35/12, A61P 31/14, C07K 14/165, C07K 14/82

(54) **ARTIFICIAL ADJUVANT VECTOR CELL CAPABLE OF INDUCING IMMUNE RESPONSE TO CORONAVIRUS, PHARMACEUTICAL COMPOSITION CONTAINING SAID CELL, AND USE APPLICATIONS OF SAID CELL AND SAID PHARMACEUTICAL COMPOSITION**

(30) Priority: 30.04.2021 JP 2021077785
(71) Applicant: RIKEN, Wako-shi, Saitama 351-0198 (JP)
(72) Inventor: FUJII, Shin-ichiro, Wako-shi, Saitama 351-0198 (JP); SHIMIZU, Kanako, Wako-shi, Saitama 351-0198 (JP)
(74) Representative: Marks & Clerk LLP
(86) International application number: PCT/JP2022/019254
(87) International publication number: WO 2022/230971

(57) **Abstract**

The present disclosure provides an artificial adjuvant vector cell inducing an immune response to a spike protein of a coronavirus, a pharmaceutical composition containing the cell, and use of the cell and the composition. According to the present disclosure, the cell can be an artificial adjuvant vector cell (aAVC) expressing a spike protein of a coronavirus.

## Description

### Technical Field

The present disclosure relates to an artificial adjuvant vector cell inducing an immune response to a coronavirus, a pharmaceutical composition containing the cell, and use of the cell and the composition.

### Background Art

Methods for inducing antigen-specific immunity in a living body have been developed as methods for preventing and treating infectious diseases and tumors. According to Patent Literature 1, when a cell of a transformant in which a target antigen and a CD1d are co-expressed is subjected to pulse treatment with a CD1d ligand, immunity specific to the target antigen can be induced in a living body.

SARS-CoV-2 binds to the ACE 2 receptor of a host cell (particularly, a type II pneumocyte) with a spike protein to infect the host cell. The spike protein has, inside thereof, a furin cleavage site that increases infectivity and pathogenicity (Non Patent Literature 1). It is disclosed that mRNA vaccines against SARS-CoV-2 currently placed on the market induce an antigen-specific antibody but cannot induce an antigen-specific T cell (see Non Patent Literature 2). On the other hand, it is disclosed that when a CTL (namely, a CD8 T cell) is induced, symptoms caused by COVID-19 can be suppressed in a subject having insufficient immunity, and that a CD8 T cell reduced the mortality rate in patients remarkably damaged in antibody responses to the B cell and SARS-CoV-2 (Non Patent Literature 3).

### Citation List

### Patent Literature

Patent Literature 1: WO2007/097370

### Non Patent Literature

Non Patent Literature 1: Andersen et al., Nature Medicine, 26, 450-452, 2020
Non Patent Literature 2: N Engl J Med. 2021 Mar 23; NEJMc2102051
Non Patent Literature 3: Res Sq., rs.3. rs-162289, 2021

### Summary of Invention

The present disclosure provides an artificial adjuvant vector cell inducing an immune response to a coronavirus, a pharmaceutical composition containing the cell, and use of the cell and the composition.

The present disclosure further provides an artificial adjuvant vector cell inducing an immune response to two different antigens, a pharmaceutical composition containing the cell, and use of the cell and the composition.

The present inventors have found that an artificial adjuvant vector cell inducing an immune response to a coronavirus can induce not only an antigen-specific antibody but also an antigen-specific immune cell (particularly, an antigen-specific T cell) in spleen and lung tissue.

The present invention provides the following inventions:
(1) A cell expressing a CD1d on a cell surface, the CD1d binding to a CD1d ligand, further expressing an antigen, wherein the antigen is a spike protein of a coronavirus or a fragment thereof, thereby capable of inducing spike protein-specific immunity (preferably in lung tissue).
(2) The cell according to (1) above, wherein the spike protein contains S1 and S2.
(3) The cell according to (1) or (2) above, further expressing an additional antigen.
(4) The cell according to (3) above, wherein the additional antigen is a cancer antigen.
(5) A composition comprising the cell according to any one of (1) to (4) above.
(6) The composition according to (5) above, for use in inducing an antigen-specific antibody and an antigen-specific T cell.
(7) The composition according to (5) above, for use in inducing an antigen-specific T cell in lung tissue.
(8) The composition according to (6) or (7) above, wherein the antigen-specific T cell is an antigen-specific cytotoxic T cell.
(9) The composition according to any one of (6) to (8) above, wherein the antigen-specific T cell is selected from the group consisting of an effector T cell and an effector memory T cell.
(10) The composition according to any one of (6) to (9) above, for single-dose administration.
(11) The composition according to any one of (6) to (10) above, for administration to a subject refractory to a vaccine containing a lipid vesicle including an mRNA encoding the spike protein in an effective amount.
(12) The composition according to any one of (6) to (11) above, for administration to a subject having received anticancer drug therapy.
(13) The composition according to (11) or (12) above, wherein the subject is a subject in which antibody production against the vaccine is not significantly induced.
(14) The composition according to (13) above, wherein the subject is a subject having a risk of increase in severity due to no significant induction of the antibody production against the vaccine.
(15) A method for inducing antigen-specific immunity in a subject, comprising:
   administering, to the subject, an effective amount of an aAVC expressing a first antigen (such as beta coronavirus S protein) and a second antigen (for example, an antigen not related to a beta coronavirus, such as a tumor-related antigen), thereby inducing antigen-specific immunity to the tumor-related antigen and beta coronavirus S protein in the subject, wherein
   the first antigen and the second antigen are different (for example, the first antigen and the second antigen being derived from different proteins of the same animal species, preferably derived from orthologs of different animal species, and more preferably derived from different proteins of different animal species), and
   the aAVC expresses a CD1d, and the CD1d is pulsed with a CD1d ligand.
(16) The method according to (15) above, wherein the subject is a subject having cancer.
(17) The method according to (15) above, wherein the subject is a subject infected with a coronavirus.
(18) The method according to (15) above, wherein the subject is a subject infected with a coronavirus, and having cancer.
(19) The method according to (15) above, comprising:
   administering, to the subject, an effective amount of the aAVC expressing the first antigen (such as beta coronavirus S protein) and the second antigen (for example, an antigen not related to beta coronavirus, such as a tumor-related antigen), thereby inducing antigen-specific immunity to the beta coronavirus S protein in lung tissue of the subject, wherein
   the aAVC expresses a CD1d, and the CD1d is pulsed with a CD1d ligand {preferably, the first antigen being a tumor-related antigen and the second antigen being beta coronavirus S protein}.
(20) The method according to (19) above, comprising:
   administering, to the subject, an effective amount of the aAVC expressing the first antigen (such as beta coronavirus S protein) and the second antigen (for example, an antigen not related to a beta coronavirus, such as a tumor-related antigen) in lung tissue (for example, in the thoracic cavity) or in a portion except for lung tissue (for example, in a vein), thereby inducing antigen-specific immunity to the beta coronavirus S protein in the lung tissue of the subject, wherein
   the aAVC expresses a CD1d, and the CD1d is pulsed with a CD1d ligand.
(21) A composition or a pharmaceutical composition, for use in the method according to any one of (15) to (20) above.

(31) The method according to any one of (15) to (20) above, wherein the first antigen (such as beta coronavirus S protein) is expressed in the cell.

(32) The method according to any one of (15) to (20) above, wherein the second antigen (such as an antigen not related to beta coronavirus, such as a tumor-related antigen) is expressed in the cell.

(33) The method according to any one of (15) to (20) above, wherein the first antigen (such as beta coronavirus S protein) and the second antigen (for example, an antigen not related to beta coronavirus S protein, such as a tumor-related antigen) are expressed in the cell.

(34) The method according to any one of (15) to (20) above, wherein the first antigen is beta coronavirus S protein, and the second antigen is a tumor-related antigen.

(35) The method according to any one of (31) to (34) above, wherein the first antigen is beta coronavirus S protein, and the second antigen is a tumor-related antigen.

(36) The method according to any one of (15) to (20) above, wherein the method can induce specific immunity to the first antigen with equivalent intensity as compared with that caused by an aAVC expressing the first antigen and not expressing or substantially not expressing the second antigen.

(37) The method according to any one of (31) to (34) above, wherein the method can induce specific immunity to the first antigen with equivalent intensity as compared with that caused by an aAVC expressing the first antigen and not expressing or substantially not expressing the second antigen.

(38) The method according to any one of (15) to (20) above, wherein the method can induce specific immunity to the second antigen with equivalent intensity as compared with an aAVC expressing the second antigen and not expressing or substantially not expressing the first antigen.

(39) The method according to any one of (31) to (34) above, wherein the method can induce specific immunity to the second antigen with equivalent intensity as compared with an aAVC expressing the second antigen and not expressing or substantially not expressing the first antigen.

(40) A composition or a pharmaceutical composition, for use in the method according to any one of (31) to (39) above.

(41) A vaccine (particularly, a coronavirus vaccine), comprising the cell according to any one of (1) to (4) above.

(42) A vaccine (particularly, a coronavirus vaccine), comprising the cell according to (4) above.

(43) A vaccine (particularly, a cancer vaccine), comprising the cell according to (4) above.

(44) A vaccine comprising the cell according to (4) above, for use in preventing or treating cancer, or for preventing or treating coronavirus infectious diseases.

### Brief Description of Drawings

[Figure 1] Figure 1 illustrates an example of production procedures for an exemplary aAVC of the present disclosure expressing a spike protein of SARS-CoV-2.
[Figure 2A] Figure 2A illustrates the relationship between the amount of mRNA introduced and the expression level of the spike protein in the exemplary aAVC.
[Figure 2B] Figure 2B illustrates expression of human CD1d (hCD1d) in the exemplary aAVC.
[Figure 2C] Figure 2C illustrates induction *in vivo* of an NKT cell by an exemplary aAVC (aAVC-CoV2) of the present disclosure expressing the spike protein of SARS-CoV-2.
[Figure 3A] Figure 3A illustrates schedule for evaluation of CTL induction and antibody (Ab) induction by administration of aAVC-CoV2.
[Figure 3B] Figure 3B illustrates induction of a specific antibody to S1 after the administration of aAVC-CoV2.
[Figure 3C] Figure 3C illustrates induction of a specific antibody to S2 after the administration of aAVC-CoV2.
[Figure 4] Figure 4 illustrates induction of a specific antibody to INF-γ producing CD8 single positive T cell after the administration of aAVC-CoV2.
[Figure 5A] Figure 5A illustrates an experimental scheme for CTL induction in the vascular system and CTL induction in lung tissue in mice after the administration of aAVC-CoV2. In this experiment, an anti-CD45 antibody is tail-vein injected on day 7 (7d) after the administration of aAVC-CoV2, the anti-CD45 antibody is bonded to CTL of the vascular system, and lung tissue is collected 5 minutes after the administration to test induction of CTL in the lung tissue.
[Figure 5B] Figure 5B illustrates data on lung tissue of an untreated mouse (naive). The lower left panel illustrates that a CD45-positive fraction corresponding to intravascular CTL obtained from the lung tissue, and a negative fraction corresponding to CTL infiltrating the lung tissue can be separated. The lower right panel illustrates expression of CD62L and expression of CD44 relating to the intravascular CTL and the infiltrating CTL.
[Figure 5C] Figure 5C illustrates data on a mouse after the administration of aAVC-CoV2. The lower left panel illustrates that a CD45-positive fraction corresponding to the intravascular CTL obtained from the lung tissue (vascular fraction; Vascular), and a negative fraction corresponding to CTL infiltrating the lung tissue (infiltration fraction; Infiltrating) can be separated. The lower right panel illustrates expression of CD62L and expression of CD44 relating to the intravascular CTL and the infiltrating CTL.
[Figure 5D] Figure 5D illustrates a ratio (%) of CD4 single positive CD44^{hi}CD62L⁻ cell, and a ratio (%) of CD8 single positive CD44^{hi}CD62L⁻ cell in each of the vascular fraction and the infiltration fraction obtained from each of the untreated mouse and the aAVC-CoV2 administered mouse.
[Figure 6A] Figure 6A illustrates an example of production procedures for an exemplary aAVC of the present disclosure expressing a cancer antigen in addition to the spike protein of SARS-CoV-2.
[Figure 6B] Figure 6B illustrates induction of IFN-γ producing CD8-positive T cell in a mouse to which an exemplary aAVC of the present disclosure expressing ovalbumin (OVA), as a model of the cancer antigen, in addition to the spike protein of SARS-CoV-2 (aAVC-OVA-CoV-2) is administered. The CD8-positive T cell induced IFN-γ production against both the S protein and the OVA.
[Figure 6C] Figure 6C illustrates change of tumor volume (mm³) in an OVA expressing tumor graft model to which aAVC-OVA-CoV-2 is administered. As a positive control, change of tumor volume in an OVA expressing tumor graft model to which aAVC forced to express only OVA (aAVC-OVA) is administered is illustrated to be compared with the former change of tumor volume.
[Figure 7A] Figure 7A illustrates an aAVC in which a TAA protein and a spike protein are co-expressed in the cell, and a CD1d pulsed with a CD1d ligand is expressed on the cell surface.
[Figure 7B] Figure 7B illustrates antigen-specific immunity induction by an aAVC in a mouse (upper panel), and an experimental scheme for verification of an anti-tumor effect (lower panel).
[Figure 7C] Figure 7C illustrates results of the antigen-specific immunity induction by an aAVC against the TAA (OVA) and the spike protein (CoV-2-S).
[Figure 7D] Figure 7D illustrates an anti-tumor effect induced by an aAVC.
[Figure 7E] Figure 7E illustrates an anti-tumor effect induced by an aAVC.

### Description of Embodiments

### <Definitions>

Herein, the term "comprising..." is used in the meaning of encompassing "consisting of..." or "containing only...". Herein, a singular form of a noun encompasses one or more of the noun unless otherwise noted.

Herein, an "artificial adjuvant vector cell" (aAVC) refers to a cell (particularly, a human cell) expressing an exogenous or endogenous CD1d, and having a CD1d ligand loaded on the CD1d on the cell surface. An aAVC can be easily produced by those skilled in the art by employing any method known in this field. Specifically, an aAVC can be obtained by culturing a CD1d expressing cell in the presence of a CD1d ligand. When the cell is a human cell, the CD1d can be a human CD1d. As the CD1d ligand, α-galactosyl ceramide (α-GalCer) can be used. Accordingly, in one preferable embodiment of the present invention, an aAVC is a human cell expressing an exogenous or endogenous CD1d, and having α-GalCer loaded on the CD1d on the cell surface.

Herein, the term "subject" is a vertebrate, and can be, for example, a mammal including a human, such as a mammal that is infected with SARS-CoV-2 (a cat, a ferret, a bat, or a pangolin). The subject can be a subject infected with SARS-CoV-2, can be an asymptomatic carrier infected with SARS-CoV-2, and can be a subject infected with SARS-CoV-2 and having an onset of COVID-19. The subject can be a subject having a possibility (risk) that he/she has been infected with SARS-CoV-2, or can be a subject having a possibility (risk) that he/she may be infected with SARS-CoV-2. The subject can be a child (such as an infant (of 1 to 6 years old), a school-age child (of 6 to 12 years old), an adolescent (of 12 years or older), and an adult (of 20 years or older). The adult can be an adult of 30 years or older, 40 years or older, 50 years or older, 60 years or older, or 70 years or older.

Herein, the term "coronavirus" refers to a virus belonging to the order *Nidovirales,* the family *Coronaviridae,* the subfamily *Orthocoronavirinae,* and is a positive-sense single-stranded RNA virus. The coronavirus has a projection of a spike protein (S protein) on the surface thereof, and has an appearance similar to corona of the sun, and hence was named as coronavirus. It causes infections of the respiratory tract including a cold in a human. Coronaviruses belonging to the subfamily *Orthocoronavirinae* are roughly divided into an alpha coronavirus, a beta coronavirus, a gamma coronavirus, and a delta coronavirus. SARS-related coronaviruses are classified into the beta coronavirus. The SARS-related coronaviruses include SARS coronavirus (SARS-CoV) and SARS coronavirus 2 (SARS-CoV-2). The SARS-CoV-2 is causing pandemic of novel coronavirus infection (COVID-19) from the end of 2019. The SARS-related coronaviruses bind to the ACE 2 receptor of a host cell with the S protein to infect the host cell. In terms of infecting a cell utilizing the ACE receptor, the SARS-related coronaviruses have common infection mechanism. The S protein of SARS-CoV-2 has, inside thereof, a furin cleavage site that increases infectivity and pathogenicity (Andersen et al., Nature Medicine, 26, 450-452, 2020).

Herein, the "SARS-CoV-2" is a coronavirus corresponding to a cause of the pandemic caused in 2020. On January 7, 2020, the World Health Organization (WHO) provisionally named this virus as 2019-nCoV. On February 11 of the same year, the International Committee on Taxonomy of Viruses (ICTV) officially named this virus as SARS-CoV-2. A coronavirus can cause diseases ranging from a general cold to severe respiratory diseases such as severe acute respiratory syndrome (SARS) and middle east respiratory syndrome (MERS). The WHO named diseases caused by this novel coronavirus as COVID-19. The International Committee on Taxonomy of Viruses deems that SARS-CoV-2 belongs to the beta coronavirus, and is the same species as (or sister taxon of) SARS-CoV. The complete genome sequence of SARS-CoV-2 is registered as GenBank Accession No: MN908947.3 in National Center for Biotechnology Information (NCBI), USA. A virus particle (virion) thereof has a particle size of about 50 to 200 nm, and contains a spike protein, a nucleocapsid protein, a membrane protein, and an envelope protein, and a virus genome RNA in the same manner as a general coronavirus. The nucleocapsid protein forms a complex together with the RNA, and the spike protein bonded to a lipid, the membrane protein, and the envelope protein surround the complex to form an envelope of the virion. It is presumed that the spike protein positioned on the outermost surface of the envelope binds to the ACE2 receptor on a cell surface to accelerate infection to the cell. There are people that do not display symptoms of the disease even when infected with SARS-CoV-2, and these people are called asymptomatic carriers. It is pointed out that asymptomatic carriers can infect others with the virus carried by them. It is pointed out that the infection with SARS-CoV-2 causes reduction or loss of smell and/or taste. SARS-CoV-2 may cause severe acute respiratory syndrome in some cases. Regarding the severe acute respiratory syndrome, fever of about 40°C, cough, and shortness of breath are reported as main symptoms. A notable complication caused thereby is pneumonia. The infection with SARS-CoV-2 is determined mainly by PCR test. In this PCR test, it is evaluated whether or not the gene of SARS-CoV-2 is present in the body depending on whether or not a band is amplified specifically to SARS-CoV-2. Examples of treatment of SARS-CoV-2 include antiviral drugs against SARS-CoV-2 (such as remdesivir), steroidal anti-inflammatory drugs (such as dexamethasone), and inflammatory cytokine inhibitors (for example, IL-6 inhibitors such as anti-IL-6 antibody, and TNF-α inhibitors such as etanercept).

Herein, the "spike protein" can be a protein encoded at positions 21563 to 25384 of the SARS-CoV-2 genome registered as GenBank Accession No: MN908947.3 in National Center for Biotechnology Information (NCBI), USA, or a protein having an amino acid sequence of SEQ ID NO: 1, and the spike protein of SARS-CoV-2 has an amino acid sequence registered as GenBank Accession No: QHD43416.1 in NCBI. The spike protein contains S1 and S2, the S1 is present at positions 13 to 541 in the amino acid sequence, and the S2 is present at positions 543 to 1208 in the amino acid sequence. The S1 further contains an N-terminal domain (NTD) and a receptor binding domain (RBD), the NTD is present at positions 13 to 304 in the amino acid sequence, and the RBD is present at positions 319 to 541. The S1 and the S2 are cut within the cell to be produced as different peptides, and form a complex in forming a virus particle. The spike protein is called also as the S protein. As the spike protein, any one of spike proteins (having an amino acid sequence corresponding to the amino acid sequence registered as GenBank Accession No: QHD43416.1 in NCBI) contained in natural viruses (for example, including mutant viruses) can be used.

Herein, the term "peptide" means a polymer of an amino acid. The polymer usually has no branch. The term "partial peptide" means a part of a specific peptide. A peptide and a partial peptide can be produced from a nucleic acid encoding the peptide. A peptide and a partial peptide can be chemically synthesized. A peptide and a partial peptide are isolated, concentrated, or purified. Isolation means that the peptide and the partial peptide are separated from at least other components, and purification means that the peptide and the partial peptide are at least selectively separated. Concentration means that the peptide and the partial peptide are increased in the density.

Herein, the term "composition" refers to a mixture of one or more components. A composition can contain, for example, a partial peptide and an aqueous solvent (such as water). The composition may further contain a pharmaceutically acceptable excipient. Herein, an immunogenic composition refers to a composition that can cause, through administration to a subject, an immune reaction in the body of the subject. An immunogenic composition can be used for inducing an immune reaction in a subject. The immunogenic composition can cause an immune reaction in a subject, and hence can be used as a vaccine. For example, an immunogenic composition of the present invention can be used for inducing an immune reaction to a SARS-related coronavirus (such as SARS-CoV-2), or can be used as a vaccine or a therapeutic agent against a SARS-related coronavirus (such as SARS-CoV-2). According to the present disclosure, an immunogenic composition containing an aAVC can induce acquired immunity such as antigen-specific antibody and/or antigen-specific cellular immunity.

Herein, the term "treatment" encompasses preventive treatment and therapeutic treatment. The therapeutic treatment can be performed on an infecting virus, and the preventive treatment can be performed in order to prevent future infection, or in order to delay onset of COVID-19 caused by future infection, or reduce symptoms of COVID-19 developed. The therapeutic treatment can be performed on a patient having symptoms or an asymptomatic carrier. The preventive treatment can be performed on an uninfected person.

Herein, the term "exogenous" is used replaceably as a term that refers to artificial introduction of a gene or nucleic acid into a target cell by genetic engineering or operation of gene transfer or the like, or refers to the gene or nucleic acid artificially introduced into the target cell, or an expressed protein thereof. An exogeneous gene can be operably linked to a promoter sequence driving the expression of the gene. Herein, the term "endogenous" means being inherent in the cell.

Herein, the term "derived" is used to indicate an animal species or protein from which a cell, a protein, or a fragment thereof is obtained. For example, a human-derived cell means that the cell is a cell obtained from a human, or is a cell line obtained by passage culture of the cell, and means that it is a human cell. A protein fragment derived from a S protein can be a fragment having a part of the amino acid sequence of the S protein.

Herein, the term "identity" means a value of Identity obtained with EMBOSS Needle (Nucleic Acids Res.; 2015; 43: W580-W584) using parameters prepared as defaults. The parameters are as follows:
Gap Open Penalty = 10
Gap Extend Penalty = 0.5
Matrix = EBLOSUM62
End Gap Penalty = false

### <aAVC of Present Disclosure, Composition Containing the aAVC, and Use Thereof>

The present disclosure provides an aAVC expressing a spike protein of a coronavirus. More specifically, the present disclosure provides a cell having a CD1d expressed on the cell surface, the CD1d binding to a CD1d ligand, further expressing an antigen, and the antigen being a spike protein of a coronavirus or a fragment thereof, thereby capable of inducing spike protein-specific immunity. In a preferable embodiment, the cell is a mammal-derived, preferably primates-derived, and more preferably human-derived cell (or a human cell). The cell is an isolated cell. The aAVC can present the CD1d bound by a CD1d ligand to an NKT cell in a living body. In a preferable embodiment, the coronavirus can be a SARS-related coronavirus (such as SARS-CoV-2).

A cell used in the present invention may be a cell derived from any human tissue such as stomach, small intestine, large intestine, lung, pancreas, kidney, liver, thymus, spleen, prostate, ovarian, uterine, bone marrow, skin, muscle, or peripheral blood as long as it has the ability to proliferate. In one embodiment, a human-derived cell used in the present invention is a non-blood cell. The cell used in the present invention may be a cell derived from a specific cell species in a tissue (such as an epithelial cell, an endothelial cell, an epidermal cell, a stromal cell, a fibroblast, an adipose tissue, a mammary gland cell, a mesangial cell, a pancreatic β cell, a nerve cell, a glial cell, an exocrine epithelial cell, an endocrine cell, a skeletal muscle cell, a smooth muscle cell, a cardiac myocyte, an osteoblast, a germ cell, or an immune cell). The cell used in the present invention may be a normal cell, or a cancer cell. In one embodiment, the cell used in the present invention is a normal cell. In one embodiment, the cell used in the present invention is human embryonic kidney cell 293 (HEK293) cell (J. Gen. Virol.; 1977; 36: 59-74), WI-38 cell, SC-01MFP cell, or MRC-5 cell, or a cell derived from any of these cells. In one embodiment, a human-derived cell used in the present invention is a cell derived from HEK293 cell. In one embodiment, the human-derived cell used in the present invention is HEK293 cell, or FreeStyle(TM) 293-F cell. Such a cell can be preferably a cell conditioned in a serum free medium. The cell used in the present invention can be, for example, an alveolar epithelial cell, particularly, a type II pneumocyte. When a type II pneumocyte is used, a S protein more similar to a S protein produced in a living body can be produced. The cell used in the present invention expresses furin in many cases, and furin may be additionally expressed. In administration to a human, the aAVC is preferably a human-derived cell.

In one embodiment, the cell used in the present invention is a tissue-derived immortalized cell or established cell line. An immortalized cell and an established cell line can be produced by those skilled in the art by known methods.

The CD1d used in the present invention may be a CD1d present in nature. The CD1d may be a CD1d endogenously expressing in a used cell, or may be a CD1d exogenously expressed in a used cell. Expression means expression in any portion of a cell in one embodiment, and means expression on a cell surface in one embodiment. In one embodiment, the aAVC expresses an exogenous CD1d. In one embodiment, the CD1d used in the present invention is a CD1d derived from a mammal (such as a human, a monkey, a mouse, a rat, a dog, or a chimpanzee). In one embodiment, the CD1d used in the present invention is a human CD1d.

In one embodiment, the human CD1d is a protein having an amino acid sequence shown in SEQ ID NO: 2. In one embodiment, the human CD1d is a protein that has an amino acid sequence including deletion, substitution, insertion, and/or addition of one to several amino acids, or in another embodiment, 1 to 10, 1 to 7, 1 to 5, 1 to 3, or 1 to 2 amino acids in the amino acid sequence shown in SEQ ID NO: 2, and has a function of a CD1d. In one embodiment, the human CD1d is a protein that has an amino acid sequence having at least 90% or more, 95% or more, 96% or more, 97% or more, 98% or more, or 99% or more identity to an amino acid sequence shown in SEQ ID NO: 4, and has a function of a CD1d.

An example of the function of a CD1d includes ability to bind to a CD1d ligand (such as α-GalCer). The ability of a CD1d to bind to a CD1d ligand can be easily evaluated by those skilled in the art by a known method. Besides, the function of a CD1d can be evaluated using, as an index, ability to activate human NKT cell by the aAVC. The ability to active human NKT cell can be evaluated by a method described in Patent Literature 1, or Example 4 of the present application.

According to the present disclosure, the aAVC can express a plurality of different antigens. According to the present disclosure, the aAVC expresses, for example, a plurality of (two or more) different peptide antigens. The peptide antigens may be encoded by one mRNA, or may be encoded by different mRNAs. The peptide antigens may be translated into one protein, or may be translated into different proteins. The peptide antigens may be linked to each other via, for example, an intracellularly cleavable linker (referred to as a cleavable linker).

The peptide antigens may be linked to each other via, for example, a linker not intercellularly cleaved (referred to as a non-cleavable linker). The intercellularly cleavable linker is not especially limited, and examples include a 2A peptide (DXEXNPGP; peptide having an amino acid sequence of SEQ ID NO: 3, such as T2A:
EGRGSLLTCGDVEENPGP (SEQ ID NO: 4), P2A:
ATNFSLLKQAGDVEENPGP (SEQ ID NO: 5), E2A:
QCTNYALLKLAGDVESNPGP (SEQ ID NO: 6), and F2A (VKQTLNFDLLKLAGDVESNPGP (SEQ ID NO: 7)), and a furin cleavage site (for example, RXXR, particularly R-X-K/R-R, such as RARR and RVRR, preferably RARR), and a peptide having an amino acid sequence corresponding to any of these. In the above, X indicates any amino acid. Examples of the amino acid include A, H, I, L, K, M, F, T, W, V, R, C, Q, G, P, Y, D, N, E, and S.

According to the present disclosure, for example, the aAVC expresses a plurality of (two or more) different peptide antigens. In one embodiment, the aAVC expresses the S1 subunit and/or the S2 subunit of the spike protein. In one embodiment, the aAVC expresses the S1 subunit and/or the S2 subunit of the spike protein, and may additionally express another antigen (such as a cancer antigen). In one embodiment, the aAVC expresses the S1 subunit and the S2 subunit of the spike protein, and these are linked to each other via a cleavable linker or a non-cleavable linker, or directly with no linker. In one embodiment, the aAVC expresses the S1 subunit and the S2 subunit of the spike protein as different peptides. According to the present disclosure, when the aAVC expresses a plurality of different peptide antigens, immune response specific to all the plurality of different peptides can be induced. This means that a virus can be treated, in measures against infectious diseases, even when virus mutation occurs, unless immunity against any of a plurality of different peptides is avoided, and thus, robust response of the aAVC to mutation can be assured. Besides, the aAVC can induce, in addition to innate immunity, both B cell immunity and T cell immunity (for example, CD4 single positive CD44^{hi}CD62L⁻ cell and CD8 single positive CD44^{hi}CD62L⁻ cell), which is contrastive to a conventional mRNA vaccine inducing only B cell immunity. Accordingly, the aAVC can be particularly useful for a subject having immunity reduced with age, and a subject having immunity reduced due to another treatment.

In one embodiment, the spike protein can be a spike protein expressed in a SARS-related coronavirus (such as SARS-CoV-2). In one embodiment, the spike protein of SARS-CoV-2 can be a spike protein registered as GenBank Accession No: MN908947.3 in National Center for Biotechnology Information (NCBI), USA, or a spike protein of SARS-CoV-2 having an amino acid sequence having 90% or more, 91% or more, 92% or more, 93% or more, 94% or more, 95% or more, 96% or more, 97% or more, 98% or more, or 99% or more identity thereto.

The aAVC is phagocyted by a dendritic cell in a living body, and a protein expressed in the cell of the aAVC is degraded into a peptide having about several amino acids to ten amino acids to be antigen-presented by the dendritic cell. Since it is such a partial peptide that is antigen-presented, a peptide to be expressed by the aAVC may be a partial peptide. Accordingly, in one embodiment, the antigen can be a part of a spike protein (such as a partial peptide) of a SARS-related coronavirus (such as SARS-CoV-2). A partial peptide having an amino acid sequence of a spike protein in position corresponding to the sequence has, in a natural variant of a spike protein of a SARS-related coronavirus (such as SARS-CoV-2), an amino acid sequence aligned in the corresponding position when it is aligned with the partial peptide of the spike protein of the SARS-related coronavirus (such as, SARS-CoV-2). In one embodiment, the partial peptide can contain a partial peptide of the S1 corresponding to the RBD, or a part thereof. In one embodiment, the partial peptide can contain a partial peptide of the S1 corresponding to a part except for the RBD of the S1 (such as, the NTD), or a part thereof. In one embodiment, the partial peptide can contain the S2 or a part thereof. The partial peptide has a length of, for example, 10 amino acids or more, 20 amino acids or more, or 30 amino acids or more.

In one embodiment, the spike protein contains the S1 and the S2. The aAVC expressing the spike protein can intracellularly express the spike protein. When the aAVC is broken by an NKT cell in a living body, and phagocyted by a dendritic cell, the dendritic cell is matured to move to lymphatic tissue, and activates the acquired immune system such as a T cell in the lymphatic tissue. Therefore, the spike protein may be extracellularly presented, but needs not be always extracellularly presented. In the aAVC, the antigen may be intracellularly expressed. In one embodiment, the antigen can be a spike protein containing the full lengths of the S1 and the S2. In one embodiment, the antigen may be glycosylated.

In one embodiment, the aAVC contains a polynucleotide encoding the CD1d, and a polynucleotide encoding the spike protein or a part thereof. The polynucleotide can be a DNA or an RNA. When the polynucleotide is a DNA, the polynucleotide encoding the CD1d and the polynucleotide encoding the spike protein or a part thereof are operably linked respectively to control sequences, and can be expressed in the aAVC. When the polynucleotide is a DNA, the polynucleotide encoding the spike protein (and the CD1d in some cases) operably linked to a control sequence can be incorporated into an expression vector. When the cell is endogenously expressed, there is no need to externally supply the CD1d. When the cell endogenously expresses or does not express the CD1d, however, a polynucleotide encoding the CD1d may be introduced into the cell. Such introduction of a polynucleotide into the cell can be performed by a method known to those skilled in the art. For example, as a method for introducing a polynucleotide into a cell, an electroporation method, a calcium phosphate precipitation method, a lipofection method, and the like are known. The polynucleotide may be introduced into the cell with a virus vector (for example, a lentivirus vector, an adenovirus vector, or an adeno-associated virus vector).

As the control sequence, for example, either of a promoter constitutively promoting expression and a promoter induced by an agent (such as tetracycline, or doxycycline) can be used. Examples of the promoter constitutively promoting expression include virus-derived promoters such as CMV (cytomegalovirus), RSV (respiratory syncytial virus), and SV40 (simian virus 40), an actin promoter, and an EF (elongation factor) 1α promoter. Examples of an inducible promoter include a tetracycline response factor (TRE3G promoter), a Cumate operator sequence, a λ operator sequence (12 × λOρ), and a heat shock promoter, which can be used in the present invention.

The expression vector can contain a start codon and a stop codon. In this case, an enhancer sequence, a noncoding region, a splicing portion, a polyadenylation site, a replicable unit or the like may be contained. Besides, the expression vector may contain a gene that can work as a marker for confirming the expression of a target gene (such as a drug resistance gene, a gene encoding a reporter enzyme, or a gene encoding a fluorescent protein).

In one embodiment, the antigen contains the S1 of the spike protein. In one embodiment, the antigen contains the S2 of the spike protein. In one embodiment, the antigen contains the S1 and the S2 of the spike protein. The S1 and the S2 of the spike protein have, between the S1 and the S2, a cleavage site of a protease present in the cell. The protease is furin in a natural spike protein.

In one embodiment, the aAVC expresses a first antigen and a second antigen, the first antigen and the second antigen are different from each other, and as compared with an aAVC that expresses the first antigen but does not express or does not substantially express the second antigen, the aAVC can induce specific immunity with equivalent intensity to the first antigen. In one embodiment, the aAVC expresses a first antigen and a second antigen, the first antigen and the second antigen are different from each other, and as compared with an aAVC that expresses the first antigen but does not express or does not substantially express the second antigen, the aAVC can induce specific immunity with equivalent intensity to the first antigen.

In one embodiment, the aAVC expresses, in addition to a first antigen (such as a spike protein), an additional antigen (a second antigen). In one embodiment, the aAVC expresses, in addition to a spike protein, an additional antigen (second antigen) in the cell (for example, within the cytoplasm). According to Examples described below, the aAVC expressing such a plurality of different antigens can induce antigen-specific immunity to the respective antigens *in vivo.* In a preferable embodiment, the aAVC expressing a plurality of different antigens can induce, as compared with an aAVC expressing only one of these antigens, antigen-specific immunity with equivalent intensity. For example, an aAVC expressing a spike protein (first antigen) and a second antigen can induce, as compared with an aAVC that expresses the spike protein (with equivalent intensity) but does not express or does not substantially express the second antigen, antigen-specific immunity with equivalent intensity to the spike protein. Besides, for example, an aAVC expressing a spike protein (first antigen) and a second antigen can induce, as compared with an aAVC that does not express or does not substantially express the spike protein but express the second antigen (with equivalent intensity), antigen-specific immunity with equivalent intensity to the second antigen.

Herein, the term "antigen-specific immunity with equivalent intensity" means that there is, between two groups to be compared, a difference in, for example, the number of antigen-specific INF-γ producing T cells (such as CD4-positive T cells or CD8-positive T cells) in the thymus of a subject having received the aAVC of only within ±50%, ±40%, ±30%, ±20%, ±10%, or ±5% (here, the difference is preferably smaller). Herein, the term "equivalent intensity" means that there is a difference in expression intensity, between two groups to be compared, of only within ±50%, ±40%, ±30%, ±20%, ±10%, or ±5% (here, the difference is preferably smaller). In one embodiment, the second antigen can be an endogenous antigen, and can be preferably an exogenous antigen. In one preferable embodiment, an aAVC expressing the second antigen contains, for example, a construct containing a gene encoding the antigen operably linked to the control sequence, and the construct can be a non-natural construct.

In one embodiment, the antigen contains a spike protein or a part thereof, and an additional antigen. The aAVC containing the spike protein or a part thereof, and the additional antigen can induce, when administered to a subject, both spike protein-specific immunity and immunity specific to the additional antigen. In one embodiment, the additional antigen can be a tumor-assosiated antigen (cancer antigen). The cancer antigen is not especially limited, and examples include MART-1/Melan-A, Mage-1, Mage-3, gp100, tyrosinase, CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hsp105, point mutation ras oncogene, point mutation p53 oncogene, and carcinoembryonic antigen (see, for example, Japanese Patent Laid-Open Nos. 2005-139118, 2004-147649, 2002-112780, and 2004-222726). An antigen of tumor in hematopoietic tissue (such as leukemia) is not especially limited, and examples include proteinase 3, WT-1, hTERT, PRAME, PML/RAR-a, DEK/CAN, cyclophilin B, TEL-MAL1, BCR-ABL, OFA-iLRP, Survivin, idiotype, Sperm protein 17, SPAN-Xb, CT-27, and MUC1.

According to the present disclosure, a composition containing the aAVC of the present disclosure is provided. The composition of the present disclosure is used for inducing antigen-specific immunity in a subject. Specifically, immunity specific to a spike protein can be induced. The composition of the present disclosure can induce a spike protein-specific antibody and a spike protein-specific T cell. Particularly, the T cell can be a cytotoxic T cell. The cytotoxic T cell has interferon γ producing ability. In the present disclosure, the spike protein-specific T cell can be an effector T cell and an effector memory T cell. In the present disclosure, the spike protein-specific T cell can be, for example, a CD4 single positive CD44^{hi}CD62L⁻ cell and a CD8 single positive CD44^{hi}CD62L⁻ cell. In the present disclosure, these T cells can be induced not only in a blood vessel but also in lung tissue. The composition containing the aAVC of the present disclosure may further contain a pharmaceutically acceptable excipient. The composition containing the aAVC of the present disclosure may contain an aAVC sterilized with radiation (such as γ rays).

According to the present disclosure, the composition containing the aAVC of the present disclosure can induce antigen-specific immunity in the lung. The induction of antigen-specific immunity in the lung by the composition containing the aAVC of the present disclosure can be attained also when the aAVC is systemically administered (for example, intravenously administered). The aAVC induces systemic immunity regardless of the administration route, and hence this suggests that immunity can be induced in the lung tissue regardless of the administration route. Therefore, the composition containing the aAVC of the present disclosure can provide a prevention or treatment effective for a coronavirus infecting an alveolar epithelial cell (such as a SARS-related coronavirus). According to the present disclosure, the composition containing the aAVC of the present disclosure can be used for preventing or treating infection with a coronavirus (such as a SARS-related coronavirus).

According to the present disclosure, the composition containing the aAVC of the present disclosure can induce an antigen-specific antibody and an antigen-specific T cell by single-dose administration. Accordingly, the composition containing the aAVC of the present disclosure can be single-dose administered. In one embodiment, the composition containing the aAVC of the present disclosure can be administered to a subject a plurality of times (for example, twice, three times, or four times). The composition is advantageous in that an antigen-specific antibody and an antigen-specific T cell can be induced even by single-dose administration. When administered a plurality of times, the antigen-specific immunity can be more strongly induced in the subject through immunity caused by the second and later doses.

According to the present disclosure, the composition containing the aAVC of the present disclosure can induce an antigen-specific T cell in addition to an antigen-specific antibody. On the contrary, mRNA vaccines against SARS-CoV-2 currently placed on the market induce an antigen-specific antibody but cannot induce an antigen-specific T cell (see, for example, N Engl J Med. 2021 Mar 23; NEJMc2102051). On the other hand, when a CTL (namely, a CD8 T cell) is induced, symptoms caused by COVID-19 can be suppressed in a subject having insufficient immunity (Res Sq., rs. 3. rs-162289, 2021). In particular, a CD8 T cell reduced the mortality rate in patients remarkably damaged in antibody responses to a B cell and SARS-CoV-2 (Res Sq., rs. 3. rs-162289, 2021). Although failure of a B cell and antibody response can cause increase in severity of the symptoms caused by COVID-19, the composition containing the aAVC of the present disclosure can be used for preventing and/or suppressing increase in severity of COVID-19 in a subject that does not substantially have a B cell and/or does not substantially show antibody response to an antigen.

According to the present disclosure, the composition containing the aAVC of the present disclosure can be administered to a subject that does not substantially have a B cell and/or does not substantially show antibody response to an antigen. In this embodiment, treatment effect owing to the antigen-specific T cell immunity induction can be expected in an infected subject.

According to the present disclosure, the composition containing the aAVC of the present disclosure can be used for inducing an antigen-specific T cell in a subject refractory to a vaccine containing a lipid vesicle including an mRNA encoding a spike protein in an effective amount.

According to the present disclosure, the composition containing the aAVC of the present disclosure can be administered to a subject in which significant antibody production against the vaccine cannot be caused. Since the composition containing the aAVC of the present disclosure can induce antigen-specific T cell immunity, a vaccine effect can be obtained through the T cell immunity induction even in such a subject. Such a subject can be a subject that is refractory to a vaccine containing a lipid vesicle including an mRNA encoding a spike protein in an effective amount, or a subject having received anticancer drug therapy.

According to the present disclosure, the composition containing the aAVC of the present disclosure contains an aAVC expressing an additional antigen (second antigen), the additional antigen is a cancer antigen, and the composition can be administered to a subject having cancer. According to the present disclosure, the composition containing the aAVC of the present disclosure contains an aAVC expressing an additional antigen, the additional antigen is a cancer antigen, and the composition can be administered to a subject having cancer for treating the cancer, and for inducing immunity to a coronavirus. The subject having cancer can be a subject having received or not having received anticancer drug therapy. In a subject having received anticancer drug therapy, the immunity is impaired or damaged by an anticancer drug in many cases. Even in such a subject, the composition containing the aAVC of the present disclosure can activate the immunity to exhibit a cancer treatment effect, and a virus vaccine or treatment effect.

According to the present disclosure, a method for inducing spike protein-specific antibody production in a subject is provided. According to the present disclosure, a method for inducing spike protein-specific T cell immunity in a subject is provided. According to the present disclosure, a method for inducing T cell immunity in lung tissue of a subject is provided. According to the present disclosure, the method includes administering an effective amount of the aAVC of the present disclosure or a composition containing the aAVC to a subject. The administration is not especially limited, and can be, for example, intravenous administration.

According to the present disclosure, a subject to be treated can be a subject not infected with a SARS-related coronavirus (such as SARS-CoV-2). When the aAVC of the present disclosure is administered to a subject not infected with a SARS-related coronavirus (such as SARS-CoV-2), antigen-specific immunity can be induced, and for example, an antigen-specific antibody and an antigen-specific T cell can be induced in the subject. Thus, a preventive effect against an infectious disease, or an effect of preventing increase in severity after onset can be expected. According to the present disclosure, the subject can be a subject infected with a SARS-related coronavirus (such as SARS-CoV-2). When the aAVC of the present disclosure is administered to a subject infected with a SARS-related coronavirus (such as SARS-CoV-2), antigen-specific T cell immunity is induced in the subject, and a preventive effect against increase in severity or a therapeutic effect can be exhibited. According to the present disclosure, the subject can be a subject that has been infected with a SARS-related coronavirus (such as SARS-CoV-2). The subject that has been infected with a SARS-related coronavirus (such as SARS-CoV-2) can be a subject that has been recovered from a disease caused by the SARS-related coronavirus (such as SARS-CoV-2) after the infection. According to the present invention, the subject can be a subject refractory to an mRNA vaccine against a SARS-related coronavirus (such as SARS-CoV-2). The mRNA vaccine has been found to induce an antigen-specific antibody, but has not been found to induce cellular immunity (particularly, T cell immunity). Accordingly, even when a subject is refractory to the mRNA vaccine, the aAVC of the present disclosure can be effective. The SARS-related coronavirus can be preferably SARS-CoV-2.

According to the present disclosure, the subject to be treated can be a patient in which B cell immunity has been reduced, or antigen-specific antibody response has been reduced. According to the present disclosure, the subject can be a patient in which T cell immunity is maintained. According to the present disclosure, the subject can be a patient in which B cell immunity has been reduced or antigen-specific antibody response has been reduced, and T cell immunity is maintained. According to the present disclosure, the subject can be a subject that does not substantially have a B cell and/or does not substantially show antibody response to an antigen. In an embodiment of the administration to such a patient in which B cell immunity has been reduced and antibody response has been reduced, a therapeutic effect or an effect of suppressing increase in severity by the antigen-specific T cell immunity induction in an infected subject can be expected. The patient in which B cell immunity has been reduced or antigen-specific antibody response has been reduced can be a patient having tumor, particularly a patient having received chemotherapy. According to the present disclosure, when either antigen-specific antibody response or antigen-specific T cell response is activated, an infection preventive or infection therapeutic effect by the aAVC of the present disclosure can be expected, which can be useful for a wide range of patients.

According to the present disclosure, the subject to be treated can be a patient having tumor. To a patient having tumor, an aAVC that expresses an antigen expressed by the tumor, and the spike protein or a part thereof can be administered. According to the present disclosure, the patient having tumor can be a patient having received chemotherapy. According to the present disclosure, the patient having tumor can be a patient in which B cell immunity has been reduced, or antigen-specific antibody response has been reduced. When it is administered to the patient having tumor, not only the tumor can be treated but also an antigen-specific antibody and an antigen-specific T cell can be induced in the subject. Therefore, a preventive effect against an infectious disease, or an effect of preventing increase in severity after onset can be expected.

According to the present disclosure, the subject to be treated can be a human patient of 60 years or older, 65 years or older, 70 years or older, 75 years or older, or 80 years or older. In such a patient, the immunity is often reduced with age. The aAVC can activate not only innate immunity but also the whole acquired immune system, and hence can be used for inducing antigen-specific immunity in a subject in which the immunity has been reduced. Besides, when either antigen-specific antibody response or antigen-specific T cell response is activated, an infection preventive or infection therapeutic effect by the aAVC of the present disclosure can be expected, which can be useful for a wide range of patients. According to the present disclosure, the subject to be treated can be a human patient of 60 years or older, 65 years or older, 70 years or older, 75 years or older, or 80 years or older, and not infected with a coronavirus. According to the present disclosure, the subject to be treated can be a human patient of 60 years or older, 65 years or older, 70 years or older, 75 years or older, or 80 years or older, and infected with a coronavirus. In an infected patient, a therapeutic effect of the disease, an effect of suppressing progression, and/or an effect of suppressing increase in severity can be exhibited owing to T cell immunity induced by the aAVC.

According to the present disclosure, use of an aAVC in manufacture of a pharmaceutical composition for preventing or treating a coronavirus is provided. The aAVC is the same as that described above. A subject to which the pharmaceutical composition is administered is the same as that described above.

According to the present disclosure, a composition for use in the method is provided. According to the present disclosure, an aAVC for use in the method is provided.

### Examples

### Example 1: Preparation of aAVC Presenting Spike Protein of SARS-CoV-2

In this example, an aAVC expressing a spike protein of a coronavirus (SARS-CoV-2) (hereinafter referred to as the "aAVC-CoV2") was attempted to be prepared.

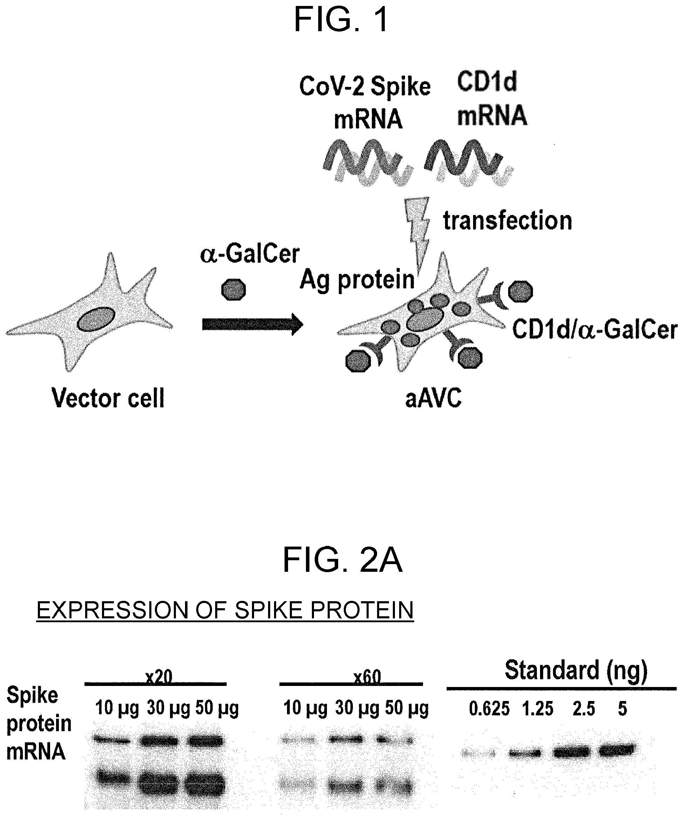

As illustrated in Figure 1, an mRNA encoding a S protein (SEQ ID NO: 1) and an mRNA encoding a CD1d (SEQ ID NO: 2) were transfected into NIH3T3 cell, and the CD1d was pulsed with a CD1d ligand to prepare an aAVC. The transfection was performed by adding the mRNA of the S protein and the mRNA of the CD1d to a cell suspension, and performing, with NEPA21 electroporator (Nepa Gene Co., Ltd.), electroporation (poring pulse: voltage of 150 V, pulse width of 8 ms, pulse interval of 50 ms, performed twice, voltage decay of 40%, and polarity of +, and transfer pulse: voltage of 20 V, pulse width of 50 ms, pulse interval of 50 ms, performed ± 5 times, voltage decay of 10%, and polarity of +/-). In this example, α-galactosyl ceramide (α-GalCer, synthesis entrusted to Juzen Chemical Corporation) was used as the CD1d ligand.

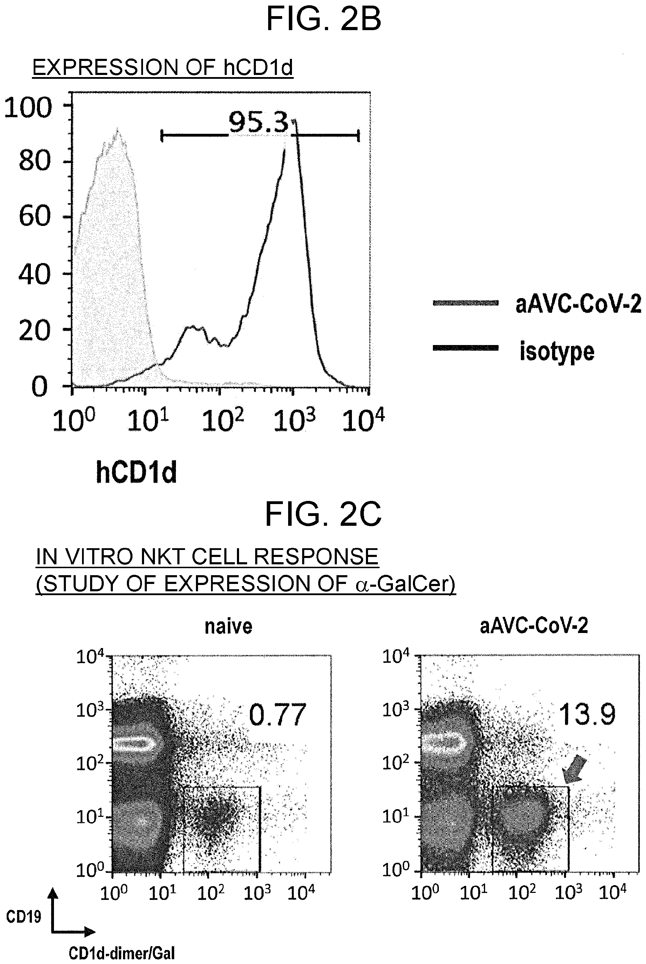

Expression of the spike protein was confirmed by Western blotting. Results were as illustrated in Figure 2A. As illustrated in Figure 2A, it was confirmed that the spike protein was expressed dose-dependently on the amount (µg) of the introduced mRNA. Besides, expression of the CD1d was measured by flowcytometry. The flowcytometry was performed using APC Mouse Anti-Human CD1d antibody (BD Biosciences, Cat. 563505) with FACS Calibur(TM) (BD Biosciences). Results were as illustrated in Figure 2B. As illustrated in Figure 2B, the expression of the CD1d was confirmed in 95.3% of cells in the aAVC.

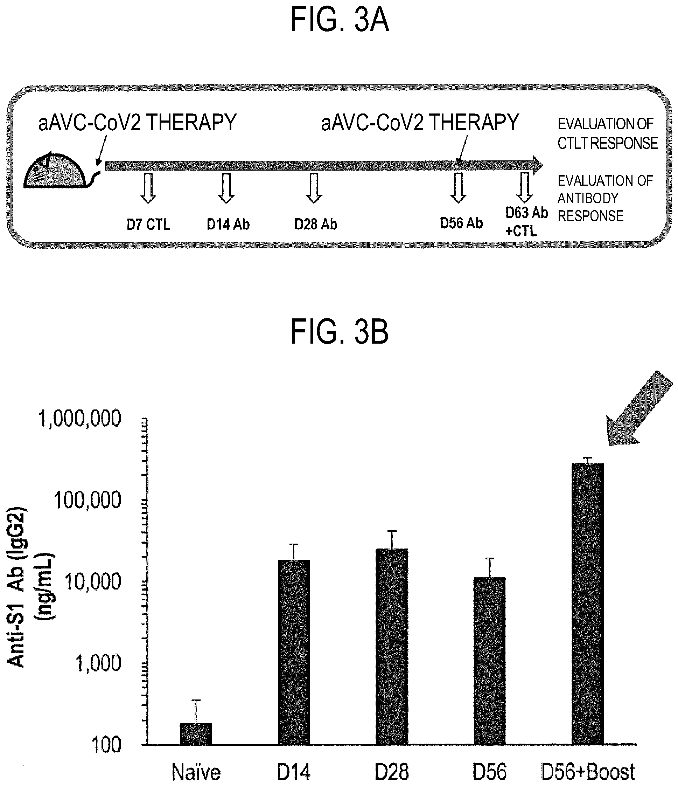

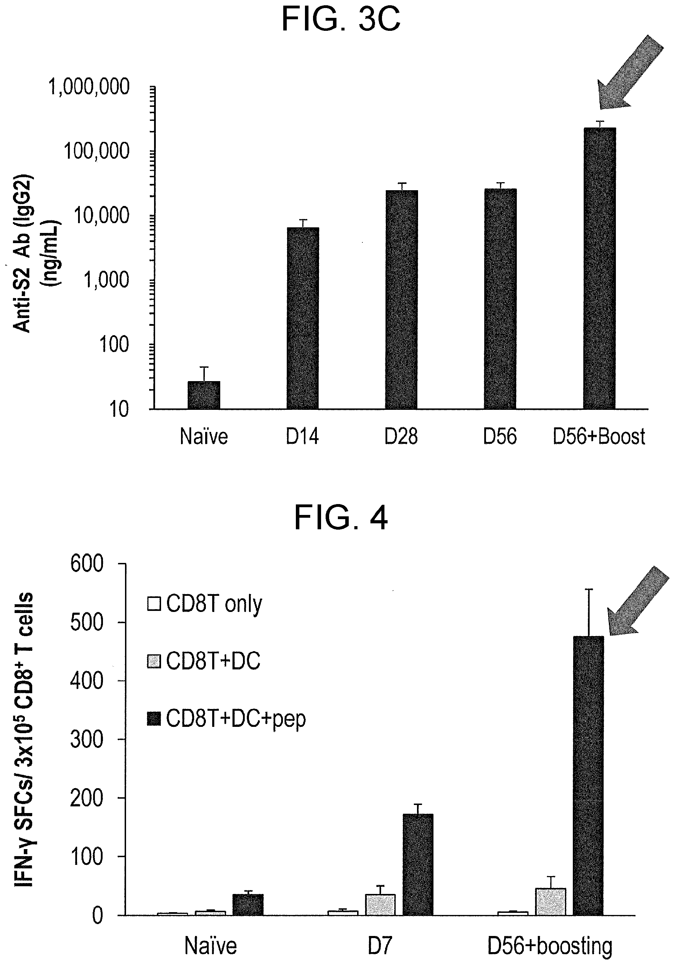

Next, phosphate buffered saline (PBS) containing the aAVC-CoV2 thus obtained was administered to a mouse via tail vein injection. The number of cells administered was 2.5 × 10⁷ cells/kg of weight. After the administration, a serum was obtained from the treated mouse in chronological order illustrated in Figure 3A, and a test of a CTL and a result of an antibody (Ab) were performed. On day 56 after the first administration, the same dose of the aAVC-CoV2 was further administered for boosting immunity. Levels of an anti-S1 antibody and an anti-S2 antibody in the serum were measured. The measurement was performed by ELISA. Results were as illustrated in Figures 3B and 3C. As illustrated in Figure 3B, the anti-S1 antibody level was dramatically increased on day 14 after the treatment, and the amount was further dramatically increased after boosting immunity. Besides, as illustrated in Figure 3C, the anti-S2 antibody level was dramatically increased on day 14 after the treatment, and the amount was further dramatically increased after boosting immunity. In this manner, it was revealed that the aAVC-CoV2 induces S1 and S2 specific immunity *in vivo.*

The level of the CTL (namely, CD8 single positive T cell, hereinafter referred to as the "CD8T cell") in the obtained spleen was measured. From the spleen, the CD8T cell was obtained with CD8 MACS Beads (Miltenyi Biotec). From the spleen of a naive mouse, a dendritic cell (hereinafter referred to as the DC) was obtained with CD11c MACS Beads (Miltenyi Biotec) as an antigen presenting cell. As an antigen, Peptivator SARS-CoV-2 Prot_S (Miltenyi Biotec) was added thereto. The amount of interferon γ (IFN-γ) produced per 3 × 10⁵ CD8T cells was measured. The measurement was performed by an ELISPOT method. Results were as illustrated in Figure 4. As illustrated in Figure 4, IFN-γ producing CTLs were induced on day 7 after the treatment, and the IFN-γ producing CTLs were further increased after boosting immunity. Thus, it was suggested that the aAVC-CoV2 causes antigen-specific T cell immunity.

The aAVC-CoV2 induced antigen-specific antibody and T cell immunity *in vivo.* This induction was enhanced by boosting immunity, which suggests that memory immunity was induced.

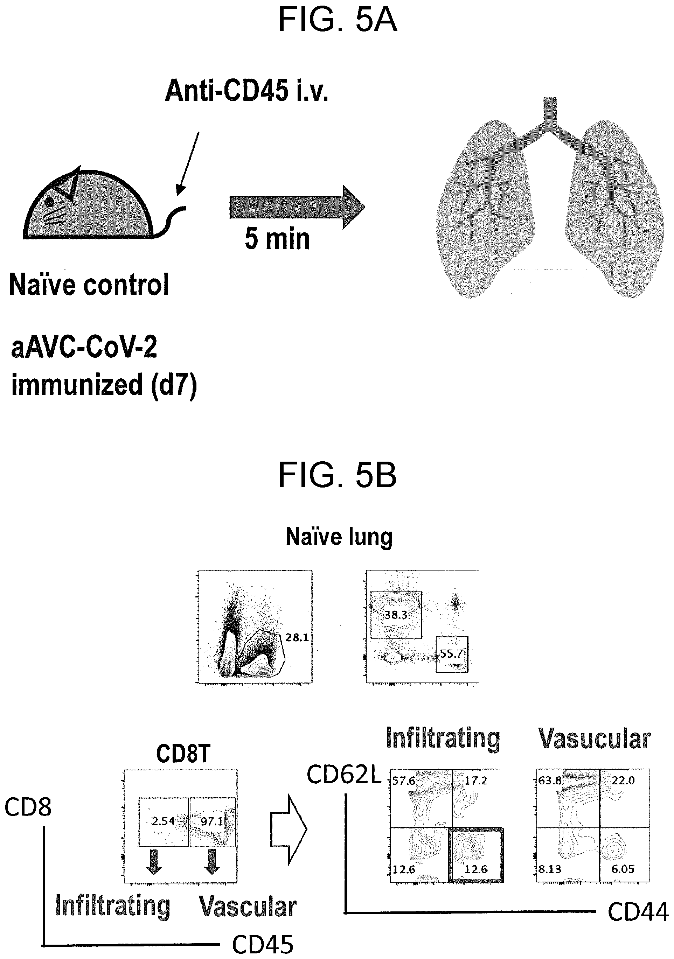

Next, the *in vivo* pharmacokinetics of the aAVC-CoV2 was confirmed. It is deemed that SARS-CoV-2 infects via a type II pneumocyte present in the lung. Therefore, CTL infiltration in the lung was confirmed. Specifically, as illustrated in Figure 5A, an anti-CD45 antibody was tail-vein injected on day 7 after the treatment with the aAVC-CoV2. The lung was collected after 5 minutes. The anti-CD45 antibody binds to blood cells (particularly, immune cells) present in the vascular system, and hence can stain these cells present in the vascular system. On the contrary, CTL infiltrating into the tissue is not stained with the antibody, and therefore, CTLs are stained, by the tail-vein injection of the anti-CD45 antibody, differently depending on positions thereof in a living body.

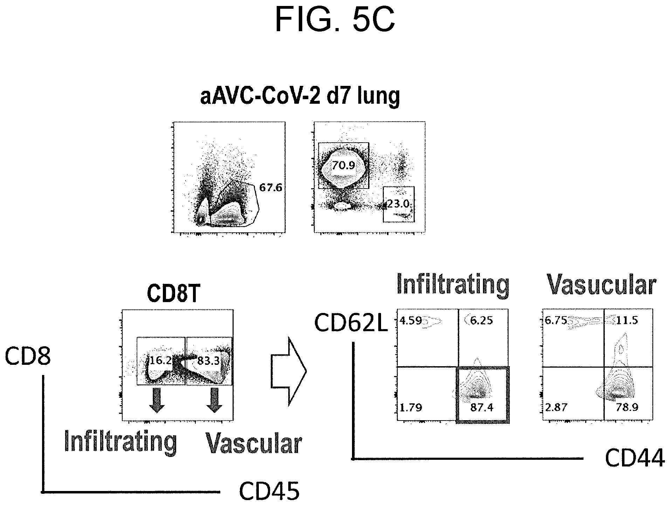

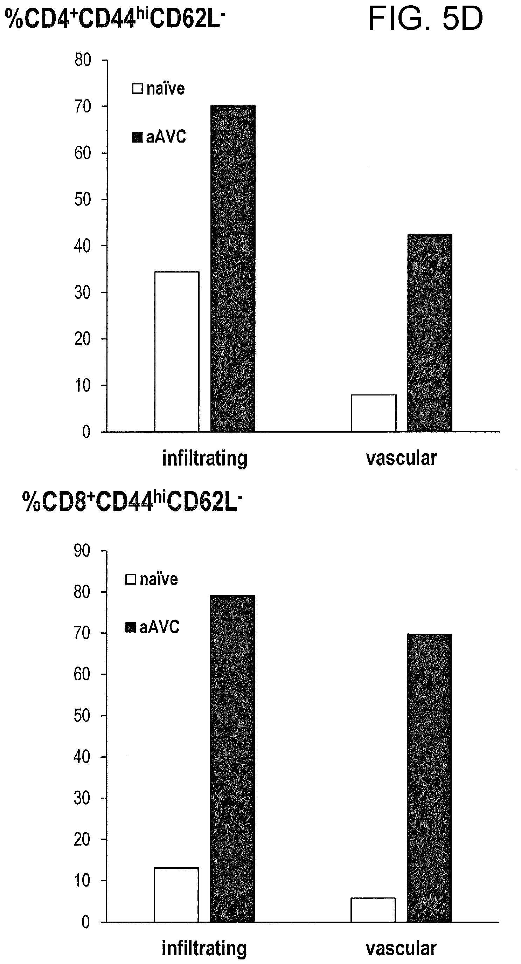

The lung was excised from the mouse on day 7 after the treatment, the resultant lung tissue was treated with collagenase D to scatter cells, and CD8-positive cells were subjected to flowcytometry based on the bond to the anti-CD45 antibody. Results were as illustrated in Figures 5B and 5C. As illustrated in Figure 5B, CTLs infiltrating into the lung tissue were only about 2.5% of the whole cells in an untreated group, but as illustrated in Figure 5C, CTLs infiltrating into the lung tissue were increased to about 16% of the whole cells in the aAVC-CoV2 treated group. Besides, a CD62L-negative CD44-positive fraction (effector T cell and effector memory cell) was only about 12.6% of the filtrated CTLs in the untreated group, but occupied 87.4% of the filtrated CTLs in the aAVC-CoV2 treated group. On the other hand, regarding CTLs present in the vascular system, a CD62L-negative CD44-positive fraction was only about 6% in the untreated group, but a CD62L-negative CD44-positive fraction occupied about 78.9% in the aAVC-CoV2 treated group. Besides, a CD4T cell obtained from the excised lung was similarly analyzed. As a result, as illustrated in Figure 5D, in the aAVC-CoV2 treated group, a ratio of a CD62L-negative CD44-positive fraction corresponding to an effector memory cell and an effector cell was increased in both the lung tissue and the vascular system.

This results from that an effector T cell having been primed and activated in the lymphatic tissue of the spleen or the like migrated from the lymphatic tissue to a blood vessel, and thereafter reached from the blood vessel to the lung tissue. It can be expected, based on this result, that the administration of the aAVC-CoV2 exhibits an antiviral effect against SARS-CoV-2 in the lung tissue.

### Example 2: Induction of Tumor-specific Immunity and Virus-specific Immunity by aAVC

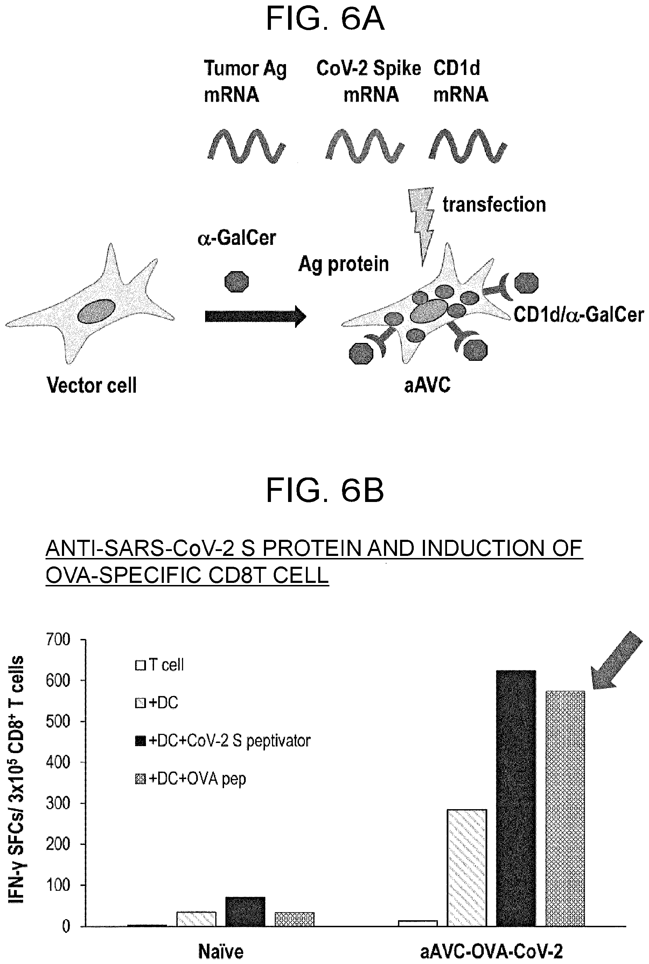

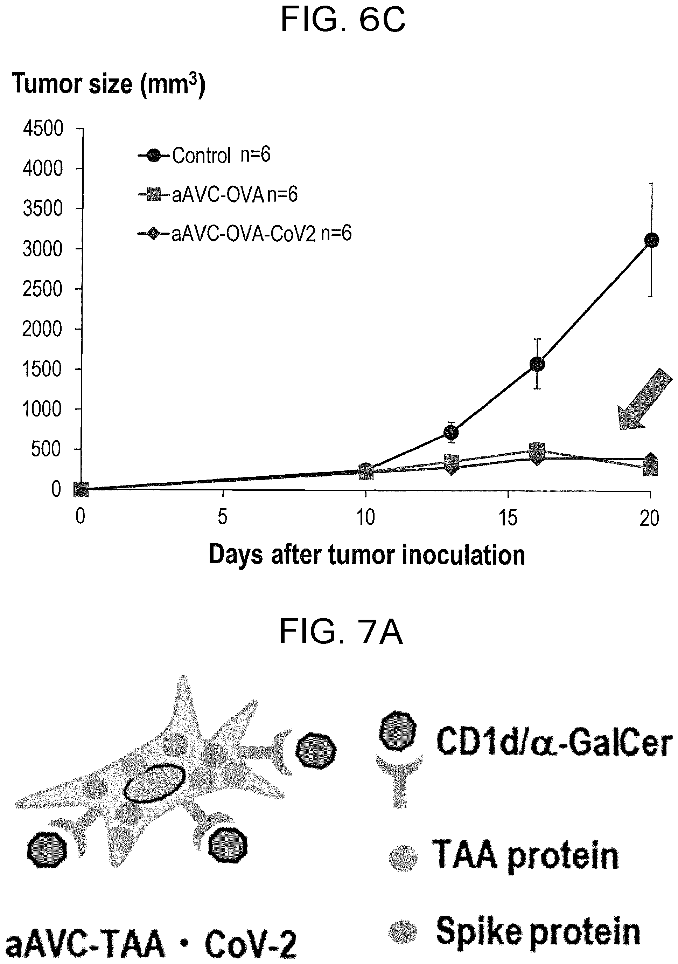

In this example, as illustrated in Figure 6A, an aAVC presenting a spike protein of SARS-CoV-2 and ovalbumin (OVA) (hereinafter referred to as the aAVC-OVA-CoV2) was prepared through the same procedures as those of Example 1, and it was tested whether or not the aAVC could induce immunity specific to a plurality of antigens. A CD8T cell was obtained in the same manner as in Example 1 to confirm IFN-γ production in the presence of a dendritic cell (DC) and an antigen peptide.

Results were as shown in Figure 6B. It was revealed that an antigen-specific CD8T cell having IFN-γ production activated was induced against OVA in an aAVC-OVA-CoV2 treated group. It was also revealed, in this group, that an antigen-specific CD8T cell having IFN-γ production activated was induced also against expression of the spike protein. In this manner, the aAVC could present a plurality of antigens, and could induce antigen-specific immunity to the plurality of antigens.

### Example 3: Antigen-specific CD8-positive T Cell Response and Antitumor Effect by Multivalent Antigen Expressing aAVC

In this example, an aAVC expressing a tumor-associated antigen (TAA) and a spike protein (aAVC-TAA/CoV-2) was produced in the same manner as in Example 1 to evaluate immunity induction ability and antitumor effect thereof.

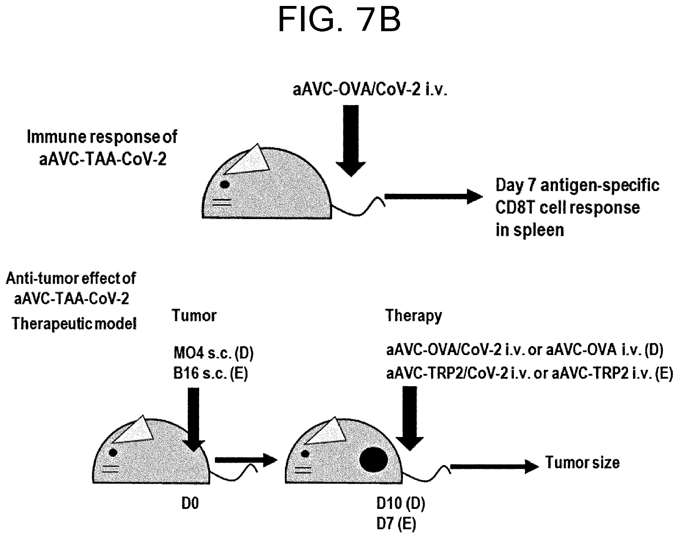

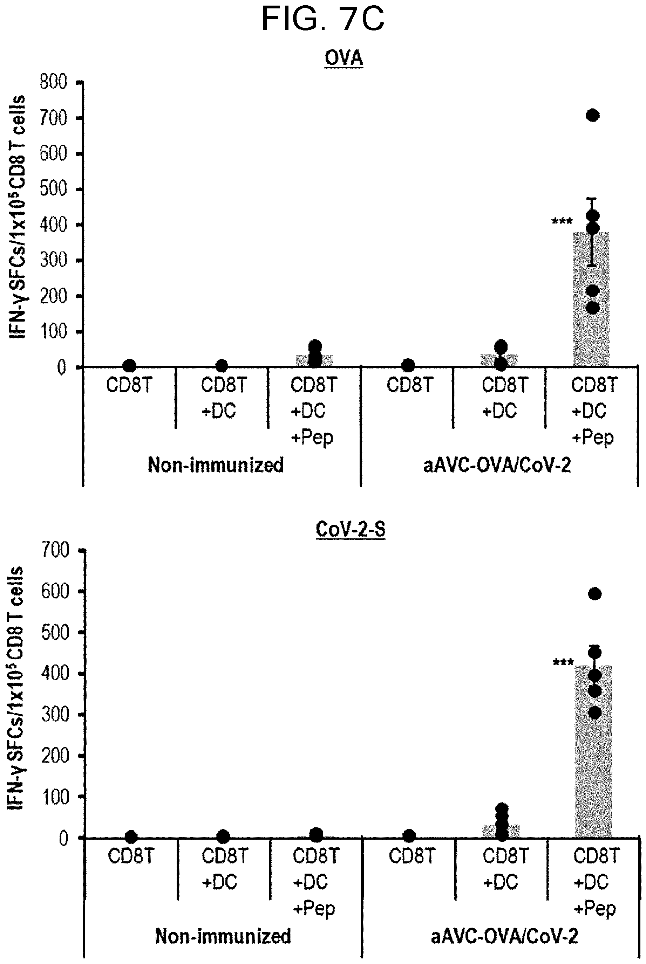

The aAVC-TAA/CoV-2 is a cell expressing TAA (OVA or TRP2) and CoV-2-S protein in the cell, and a CD1d/α-GalCer complex on the cell surface (Figure 7A). Figure 7B illustrates a test of CD8-positive T cell response (upper panel) and a test of antitumor effect (lower panel). A CD8T cell was obtained in the same manner as in Example 1, and IFN-γ production in the presence of a dendritic cell (DC) and an antigen peptide was confirmed. Specifically, 5 × 10⁵ aAVC-OVA/CoV-2 was intravenously administered to a C57BL/6J mouse. On day 7 after culturing in the protein or peptide pool for 24 hours, OVA-specific CD8+ T (upper panel of Figure 7C) and CoV-2-S specific CD8+ T (lower panel of Figure 7C) cell responses were evaluated by IFN-γ ELISPOT assay (average ± SEM, n = 5). Results were as illustrated in Figure 7C. A gray bar indicates an average, and other symbols indicate respective data (***p < 0.001 Tukey's test). As illustrated in Figure 7C, it was revealed that an antigen-specific CD8T cell having IFN-γ production activated was induced against OVA in an aAVC-OVA-CoV2 treated group, and that an antigen-specific CD8T cell having IFN-γ production activated was induced against CoV-2-S protein.

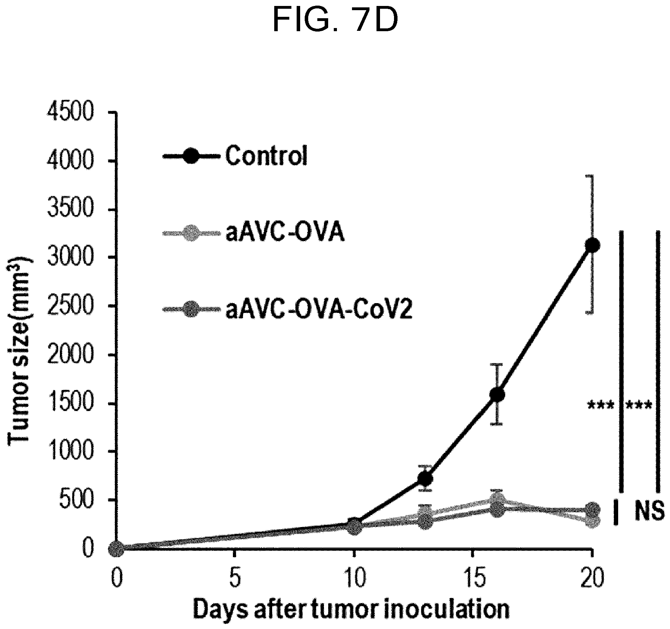

Next, an antitumor effect by the aAVC-OVA/CoV-2 was evaluated. A mouse was s.c. inoculated with 5 × 10⁵ MO4, and on day 10, 5 × 10⁵ aAVC-OVA/CoV-2 or aAVC-OVA was i.v. administered. Thereafter, the size of the inoculated tumor was measured over time (average ± SEM, n = 6 to 7/group). Results were as illustrated in Figure 7D. As illustrated in Figure 7D, the aAVC-OVA/CoV-2 exhibited an antitumor effect against MO4, and the effect was equivalent to that of the aAVC-OVA. In other words, there was no negative influence on the effect when the S protein of CoV-2 was used together as multiple antigens (**p < 0.001 Tukey's test, NS: not significant).

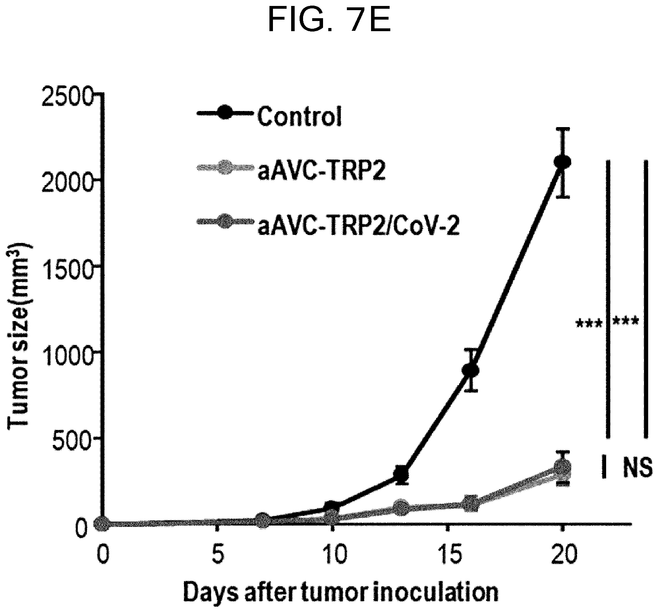

Furthermore, an antitumor effect by the aAVC-TRP2/CoV-2 was evaluated. A mouse was s.c. inoculated with 1 × 10⁵ B16, and on day 7, 5 × 10⁵ aAVC-TRP2/CoV-2 or aAVC-TRP2 was i.v. administered. Thereafter, the size of the inoculated tumor was measured over time (average ± SEM, n = 6 to 7/group). Results were as illustrated in Figure 7E. As illustrated in Figure 7E, the aAVC-TRP2/CoV-2 exhibited an antitumor effect against B16, and the effect was equivalent to that of the aAVC-TRP2. In other words, there was no negative influence on the effect when the S protein of CoV-2 was used together as multiple antigens (***p < 0.001 Tukey's test, NS: not significant).

In this manner, the aAVC expressing a plurality of different antigens can induce antigen-specific immunity to the respective antigens *in vivo.* Besides, the aAVC expressing a plurality of different antigens can exhibit an immunity induction effect comparable to that of an aAVC expressing a single antigen.

### Sequence List:

SEQ ID NO: 1: an example of an amino acid sequence of the S protein of SARS-CoV-2
SEQ ID NO: 2: an example of an amino acid sequence of human CD1d
SEQ ID NO: 3: an example of an amino acid sequence of 2A peptide
SEQ ID NO: 4: an example of an amino acid sequence of T2A peptide
SEQ ID NO: 5: an example of an amino acid sequence of P2A peptide
SEQ ID NO: 6: an example of an amino acid sequence of E2A peptide
SEQ ID NO: 7: an example of an amino acid sequence of F2A peptide

## Claims

1. A cell expressing a CD1d on a cell surface, wherein the CD1d is bound by a CD1d ligand, and further expresses an antigen, wherein the antigen is a spike protein of a coronavirus or a fragment thereof, thereby capable of inducing spike protein-specific immunity.

2. The cell according to claim 1, wherein the spike protein contains S1 and S2.

3. The cell according to claim 1 or 2, further expressing an additional antigen.

4. The cell according to claim 3, wherein the additional antigen is a cancer antigen.

5. A composition comprising the cell according to any one of claims 1 to 4.

6. The composition according to claim 5, for use in inducing an antigen-specific antibody and an antigen-specific T cell.

7. The composition according to claim 5, for use in inducing an antigen-specific T cell in lung tissue.

8. The composition according to claim 6 or 7, wherein the antigen-specific T cell is an antigen-specific cytotoxic T cell.

9. The composition according to any one of claims 6 to 8, wherein the antigen-specific T cell is selected from the group consisting of an effector T cell and an effector memory T cell.

10. The composition according to any one of claims 6 to 9, for single-dose administration.

11. The composition according to any one of claims 6 to 10, for administration to a subject refractory to a vaccine containing a lipid vesicle including an mRNA encoding the spike protein in an effective amount.

12. The composition according to any one of claims 6 to 11, for administration to a subject having received anticancer drug therapy.

13. The composition according to claim 11 or 12, wherein the subject is a subject in which antibody production against the vaccine is not significantly induced.

14. The composition according to claim 13, wherein the subject is a subject having a risk of increase in severity due to no significant induction of the antibody production against the vaccine.

15. A method for inducing antigen-specific immunity in a subject, comprising:
administering, to the subject, an effective amount of an aAVC expressing beta coronavirus S protein (first antigen) and a second antigen, thereby inducing antigen-specific immunity to the tumor-related antigen and beta coronavirus S protein in the subject, wherein
the second antigen is derived from a protein different from the first antigen, and
the aAVC expresses a CD1d, and the CD1d is pulsed with a CD1d ligand.

16. The method according to claim 15, wherein the subject is a subject having cancer.

17. The method according to claim 15, wherein the subject is a subject infected with a coronavirus.

18. The method according to claim 15, wherein the subject is a subject infected with a coronavirus, and having cancer.

19. The method according to claim 15, comprising:
administering, to the subject, an effective amount of the aAVC expressing the beta coronavirus S protein (first antigen) and the second antigen, thereby inducing antigen-specific immunity to the beta coronavirus S protein in lung tissue of the subject, wherein
the aAVC expresses a CD1d, and the CD1d is pulsed with a CD1d ligand.

20. The method according to claim 19, comprising:
intravenously administering, to the subject, an effective amount of the aAVC expressing the beta coronavirus S protein (first antigen) and the second antigen, thereby inducing antigen-specific immunity to the beta coronavirus S protein in the lung tissue of the subject, wherein
the aAVC expresses a CD1d, and the CD1d is pulsed with a CD1d ligand.

21. The method according to any one of claims 15 to 20, wherein the second antigen is a tumor-related antigen.

22. A composition or a pharmaceutical composition, for use in the method according to any one of claims 15 to 21.
